# EUROPÄISCHE PATENTSCHRIFT

(11) **EP 2 825 248 B1**
(45) Veröffentlichungstag und Bekanntmachung des Hinweises auf die Patenterteilung: **09.03.2016**
(21) Anmeldenummer: 13720225.5
(22) Anmeldetag: 14.03.2013
(51) Int. Cl.: A61M 39/10, A61M 39/20, A61M 1/16

(54) **MEDIZINISCHE VORRICHTUNG MIT EINER BUCHSEN-EINHEIT ZUM ANSCHLUSS EINER VORRICHTUNG ZUR BEREITSTELLUNG VON MEDIZINISCHEN FLÜSSIGKEITEN**
MEDICAL DEVICE COMPRISING A SOCKET UNIT FOR CONNECTING A DEVICE FOR PROVIDING MEDICAL LIQUIDS
DISPOSITIF MÉDICAL DOTÉ D'UNE UNITÉ CONNECTEUR FEMELLE POUR LE RACCORDEMENT D'UN DISPOSITIF DE FOURNITURE DE LIQUIDES MÉDICINAUX

(30) Priorität: 16.03.2012 DE 102012005189; 16.03.2012 US 201261611637 P
(43) Veröffentlichungstag der Anmeldung: 21.01.2015
(73) Patentinhaber: Fresenius Medical Care Deutschland GmbH, 61352 Bad Homburg (DE)
(72) Erfinder: BRANDL, Matthias, 97631 Bad Königshofen (DE); FAULHABER, Thomas, 97493 Bergrheinfeld (DE); HÖRMANN, Jörn, 66386 St. Ingbert (DE); KUGELMANN, Franz, 66606 St. Wendel/Bliesen (DE); ÖRTER, Gökhan, 35619 Braunfels (DE); STERZER, Rafael, 97422 Schweinfurt (DE)
(74) Vertreter: Oppermann, Frank
(86) Internationale Anmeldenummer: PCT/EP2013/000774
(87) Internationale Veröffentlichungsnummer: WO 2013/135388

(56) Entgegenhaltungen:
- WO-A1-2009/074588
- WO-A1-2009/081196

## Beschreibung

Die Erfindung betrifft eine medizinische Vorrichtung mit einer Buchsen-Einheit zum Anschluss einer Stecker-Einheit einer Vorrichtung zur Bereitstellung von medizinischen Flüssigkeiten, wobei die medizinische Vorrichtung insbesondere eine extrakorporale Blutbehandlungsvorrichtung, beispielsweise extrakorporale Dialysevorrichtung oder Vorrichtung zur Peritonealdialyse, oder eine Vorrichtung zum Befüllen oder Entleeren der Vorrichtung zur Bereitstellung von medizinischen Flüssigkeiten ist.

Zum Anschluss von externen Komponenten an medizintechnische Einrichtungen sind unterschiedliche Konnektoren bekannt. Der Zugang zu den medizintechnischen Einrichtungen erfolgt im Allgemeinen mittels Steckern, die in passende Buchsen der medizintechnischen Einrichtungen eingesteckt werden. Insofern verfügen die medizintechnischen Einrichtungen, die nachfolgend allgemein als medizinische Vorrichtungen bezeichnet werden, über eine entsprechende Buchsen-Einheit, während die externen Komponenten eine Stecker-Einheit aufweisen.

Zur Behandlung von nierenkranken Patienten finden Blutbehandlungsvorrichtungen Verwendung, zu denen insbesondere die bekannten extrakorporalen Dialysevorrichtungen oder Vorrichtungen zur Peritonealdialyse gehören. Zur Blutreinigung des Patienten ist die Bereitstellung von medizinischen Behandlungsflüssigkeiten erforderlich. Zu diesen zählen beispielsweise die Dialysierflüssigkeit oder Substitutionsflüssigkeit. In der sogenannten Automatic Peritoneal Dialysis (APD) oder der Akutdialyse werden die medizinischen Behandlungsflüssigkeiten in den Blutbehandlungsvorrichtungen automatisch verarbeitet. Die Behandlungsflüssigkeiten werden in Flüssigkeitsreservoirs bereitgestellt, die an die Blutbehandlungsvorrichtungen angeschlossen werden.

Die frische Dialysierflüssigkeit wird aus dem Flüssigkeitsreservoir in die Blutbehandlungsvorrichtung gepumpt. Das Flüssigkeitsreservoir kann bereits ein Konzentrat enthalten, das mit Wasser verdünnt wird. In diesem Fall muss das Flüssigkeitsreservoir nur mit Wasser befüllt werden. Daher wird in diesem Zusammenhang auch Wasser als medizinische Flüssigkeit verstanden. Es ist auch möglich, dass mehrere Flüssigkeitsreservoirs mit einer Blutbehandlungsvorrichtung verbunden sind, wenn in der Behandlungsvorrichtung eine gebrauchsfertige Behandlungsflüssigkeit durch Mischen mehrerer Flüssigkeiten hergestellt wird. Der Anschluss des Flüssigkeitsreservoirs an die Blutbehandlungsvorrichtungen erfolgt wieder mit einer Stecker-Einheit, die in eine Buchsen-Einheit der Blutbehandlungsvorrichtung eingesteckt wird.

Zum Befüllen der Vorrichtungen zur Bereitstellung von Dialysierflüssigkeit sind Vorrichtungen bekannt, an die sich die Vorrichtungen zur Bereitstellung von Dialysierflüssigkeit anschließen lassen. Dafür verfügen die Vorrichtungen zum Befüllen wiederum über eine Buchsen-Einheit, die sich mit der Stecker-Einheit der Vorrichtung zur Bereitstellung von Dialysierflüssigkeit konnektieren lässt.

Eine Vorrichtung zur Bereitstellung einer Behandlungsflüssigkeit ist beispielsweise aus der EP 0 575 970 A2 bekannt. Die bekannte Vorrichtung zur Bereitstellung von Dialysierflüssigkeit umfasst einen Beutel zur Aufnahme der Flüssigkeit, an dem eine Schlauchleitung angeschlossen ist, die an ihrem freien Ende mit einem Stecker verbunden ist. Die Dialysevorrichtung verfügt über eine Buchse, in die der Stecker eingesteckt wird. Mit dem Stecker und der Buchse können zwei Strömungsverbindungen hergestellt werden, um frische Dialysierflüssigkeit aus dem Beutel in die Dialysevorrichtung und verbrauchte Dialysierflüssigkeit zurück in den Beutel leiten zu können. Zur Sicherung des Steckers in der Buchse gegen Herausrutschen weist der Stecker Rastnasen auf, die in Ausnehmungen der Buchse greifen, wenn der Stecker vollständig in die Buchse eingesteckt ist.

Der Anschluss der Vorrichtung zur Bereitstellung von medizinischen Flüssigkeiten an die Blutbehandlungsvorrichtung oder die Vorrichtung zum Befüllen der Vorrichtung zur Bereitstellung von medizinischen Flüssigkeiten soll für das medizinische Personal möglichst einfach und sicher sein. Zur Desinfektion sollte sich die Buchsen-Einheit mit einer Spülflüssigkeit spülen lassen.

Aus der WO 2009/074588 A1 ist eine Buchsen-Einheit für eine Dialysevorrichtung bekannt, die mit einer Spülflüssigkeit gespült werden kann. Die Buchse der Buchsen-Einheit weist einen zylindrischen Gehäusekörper auf, in dem ein Anschlussstück für einen Konnektor einer Stecker-Einheit angeordnet ist. Zum Spülen wird der Gehäusekörper der Buchsen-Einheit von einem Verschlussstück verschlossen, so dass die zylindrische Ausnehmung in dem Gehäusekörper eine Spülkammer bildet, die von der Spülflüssigkeit durchströmt wird. Das Verschlussstück ist an dem Gehäusekörper der Buchseneinheit zwischen einer ersten Position, in der die Spülkammer verschlossen ist, und einer zweiten Position, in der die Spülkammer offen ist, verschiebbar geführt.

Der Erfindung liegt die Aufgabe zugrunde, die Versorgung von medizinischen Vorrichtungen, insbesondere Blutbehandlungsvorrichtungen, beispielsweise extrakorporale Dialysevorrichtungen oder Vorrichtungen zur Peritonealdialyse, mit medizinischen Flüssigkeiten für das medizinische Personal zu vereinfachen und die Sicherheit der Behandlung zu erhöhen.

Die Lösung dieser Aufgabe erfolgt erfindungsgemäß mit den Merkmalen des Patentanspruchs 1. Vorteilhafte Ausfiihrungsformen der Erfindung sind Gegenstand der Unteransprüche.

Die erfindungsgemäße medizinische Vorrichtung zur Verarbeitung medizinischer Flüssigkeiten verfügt über eine Buchsen-Einheit, während die erfindungsgemäße Vorrichtung zur Bereitstellung von medizinischen Flüssigkeiten über eine Stecker-Einheit verfügt.

Die Buchsen-Einheit und die Stecker-Einheit zeichnen sich dadurch aus, dass sich mit beiden Einheiten eine Strömungsverbindung zwischen der medizinischen Vorrichtung einerseits und der Vorrichtung zur Bereitstellung von medizinischen Flüssigkeiten andererseits einfach und sicher herstellen lässt.

Zur Herstellung der Strömungsverbindung verfügt die Buchsen-Einheit über mindestens ein Anschlussstück, während die Stecker-Einheit über mindestens einen Konnektor verfügt, so dass sich eine flüssigkeitsdichte Verbindung herstellen lässt, wenn das Anschlussstück an den Konnektor angeschlossen wird. Unerheblich ist, wie Anschlussstücke und Konnektoren ausgebildet sind. Anschlussstück und Konnektor können ihrerseits Stecker bzw. Buchsen sein.

Zum Zuführen von beispielsweise frischer Behandlungsflüssigkeit und Abführen von beispielsweise verbrauchter Behandlungsflüssigkeit können zur Herstellung mehrerer Strömungsverbindungen auch mehrere Anschlussstücke und Konnektoren vorgesehen sein.

Bei der erfindungsgemäßen Buchsen-Einheit ist das mindestens eine Anschlussstück unter Bildung einer Spülkammer von einem Anschlussteil konzentrisch umschlossen, wobei die Buchsen-Einheit einen Verschlusskörper mit mindestens einem Verschlussstück zum Verschluss der mindestens einen Spülkammer aufweist.

Das Grundprinzip der Erfindung liegt darin, dass der Verschlusskörper um eine Drehachse drehbar gelagert ist, wobei das mindestens eine Verschlussstück an dem Verschlusskörper im Abstand zu der Drehachse angeordnet ist. Der Verschlusskörper kann zwischen einer ersten und einer zweiten Drehstellung verdreht werden.

In der ersten Drehstellung liegen das mindestens eine Verschlussstück und der mindestens eine Anschlussteil auf einer gemeinsamen Achse, so dass durch eine Relativbewegung von Stecker-Einheit und Buchsen-Einheit eine Verbindung zwischen Verschlussstück und Anschlussteil zum Verschluss der Spülkammer herstellbar ist. In diesem Zusammenhang wird unter einer Relativbewegung von Verschlussstück und Anschlussteil eine Bewegung eines verschiebbaren Verschlussstücks auf einen feststehenden Anschlussteil oder eine Bewegung eines verschiebbaren Anschlussteils auf ein feststehendes Verschlussstück verstanden. Mit der Relativbewegung von Verschlussstück und Anschlussteil kann ein sicherer Verschluss der Spülkammer erreicht werden, in dem Verschlussstück und Anschlussteil ineinandergreifen können.

In der zweiten Drehstellung sind das mindestens eine Verschlussstück und der mindestens eine Anschlussteil bzw. das Anschlussstück zueinander versetzt angeordnet, so dass beim Einstecken der Stecker-Einheit in die Buchsen-Einheit eine Verbindung zwischen dem mindestens einen Konnektor der Stecker-Einheit und dem mindestens einen Anschlussstück der Buchsen-Einheit herstellbar ist.

Bei einer bevorzugten Ausführungsform weist die Buchsen-Einheit Mittel zum Vorschieben des mindestens einen Anschlussteils bzw. Anschlussstücks aus der Buchsen-Einheit und zum Zurückziehen des mindestens einen Anschlussteils bzw. Anschlussstücks in die Buchsen-Einheit auf. Es ist aber auch möglich, dass der Anschlussteil feststeht und das Verschlussstück verschiebbar ist.

Bei einer besonders bevorzugten Ausfuhrungsform verfügt die Buchsen-Einheit über ein erstes Anschlussstück zum Anschluss eines ersten Konnektors der Stecker-Einheit und ein zweites Anschlussstück zum Anschluss eines zweiten Konnektors der Stecker-Einheit, so dass eine erste Strömungsverbindung zum Zuführen von Flüssigkeit und eine zweite Strömungsverbindung zum Abführen von Flüssigkeit herstellbar ist, während die Buchsen-Einheit einen Verschlusskörper mit einem ersten Verschlussstück zum Verschluss der ersten Spülkammer und einem zweiten Verschlussstück zum Verschluss der zweiten Spülkammer der Buchsen-Einheit aufweist, wobei das erste und zweite Verschlussstück zu beiden Seiten der Drehachse angeordnet sind.

Der Verschlusskörper ist vorzugsweise derart ausgebildet, dass in der ersten Drehstellung die mindestens eine Spülkammer mit dem Verschlussstück verschlossen werden kann und in der zweiten Drehstellung der mindestens eine Konnektor der Stecker-Einheit mit dem mindestens einen Anschlussstück der Buchsen-Einheit verbunden werden kann. Eine weitere besonders bevorzugte Ausführungsform sieht daher eine Ausbildung des Verschlusskörpers derart vor, dass der Verschlusskörper zu beiden Seiten der Drehachse angeordnete Ausschnitte zur Aufnahme der Konnektoren der Stecker-Einheit beim Einstecken der Stecker-Einheit in die Buchsen-Einheit aufweist. Der zwischen den Ausschnitten und den Konnektoren eingeschlossene Winkel ist vorzugsweise ein rechter Winkel.

Eine weitere besonders bevorzugte Ausführungsform sieht eine automatische Konnektion von Anschlussstück und Konnektor. Es ist daher nicht erforderlich, die Stecker-Einheit vollständig in die Buchsen-Einheit einzustecken. Es genügt die Stecker-Einheit lose in die Buchsen-Einheit einzusetzen. Die flüssigkeitsdichte Konnektion von Anschlussstück und Konnektor erfolgt dann automatisch.

Die Buchsen-Einheit verfügt vorzugsweise über Mittel zum lösbaren Verbinden der Stecker-Einheit an der Buchsen-Einheit, die derart ausgebildet sind, dass bei der Ausführung der Relativbewegung zwischen dem mindestens einen Anschlussstück der Buchsen-Einheit und dem mindestens einen Konnektor der Stecker-Einheit die zunächst nur lose in die Buchsen-Einheit eingesetzte Stecker-Einheit fest mit der Buchsen-Einheit verbunden wird.

Die Mittel zum lösbaren Verbinden der Stecker-Einheit und der Buchsen-Einheit weisen vorzugsweise ein Aufnahmestück auf, in das ein Ansatzstück der Stecker-Einheit passend eingesetzt werden kann. Das Aufnahmestück der Buchsen-Einheit ist vorzugsweise als ein rohrförmiger Körper ausgebildet ist, in den das Ansatzstück der Stecker-Einheit einsetzbar ist.

Vorzugsweise weist das Aufnahmestück der Buchsen-Einheit Ausnehmungen zur Aufnahme von Rastnasen des Ansatzstücks der Stecker-Einheit auf. Dadurch kann eine einrastende Verbindung hergestellt werden. Damit sind Aufnahmestück und Ansatzstück aber noch nicht verriegelt.

Zur Verriegelung des Ansatzstücks in dem Aufnahmestück weisen die Mittel zum lösbaren Verbinden der Stecker-Einheit und der Buchsen-Einheit vorzugsweise einen stiftförmigen Körper auf, der in das Ansatzstück der Stecker-Einheit einführbar ist, sodass zur Herstellung einer festen Verbindung zwischen Stecker- und Buchsen-Einheit das Ansatzstück der Stecker-Einheit aufgespreizt wird. Damit ist das Ansatzstück der Stecker-Einheit in dem Aufnahmestück der Buchsen-Einheit gegen Herausrutschen gesichert. Die Verriegelung von Ansatzstück und Aufnahmestück erfolgt bei der besonders bevorzugten Ausführungsform dadurch, dass mit dem Aufspreizen des Ansatzstücks die Rastnasen in Ausnehmungen des Aufnahmestücks gegen Herausrutschen gesichert werden.

Eine weitere besonders bevorzugte Ausführungsform der Erfindung sieht die automatische Erkennung des Ansatzstücks der Stecker-Einheit in dem Aufnahmestück der Buchsen-Einheit vor. Die Mittel zum Detektieren des Ansatzstücks in dem Aufnahmestück weisen vorzugsweise ein federnd vorgespanntes Tastelement auf, das derart in der Buchsen-Einheit angeordnet ist, dass das Tastelement beim Einführen des Ansatzstücks der Stecker-Einheit in das Aufnahmestück der Buchsen-Einheit entgegen einer Federspannung verschoben wird. Die Verschiebung des Tastelements kann mit bekannten Mitteln erkannt werden. Beispielsweise können hierzu elektrische Kontakte, die beim Verschieben des Tastelements geschlossen bzw. geöffnet werden, oder eine Lichtschranke vorgesehen sein, mit der die Stellung des Tastelements detektiert wird.

Eine besonders bevorzugte Ausführungsform sieht vor, dass das rohrförmige Aufnahmestück drehbar an der Buchsen-Einheit gelagert ist, wobei der Verschlusskörper an dem Aufnahmestück der Buchsen-Einheit befestigt ist. Dadurch wird eine Einheit mit einem besonders kompakten Aufbau geschaffen.

Im Folgenden wird ein Ausführungsbeispiel der Erfindung unter Bezugnahme auf die Figuren näher erläutert.

Es zeigen:
- Fig. 1:: eine Vorrichtung zur Bereitstellung einer medizinischen Flüssigkeit, insbesondere Dialysierflüssigkeit, zusammen mit einer Blutbehandlungsvorrichtung und einer Vorrichtung zum Befüllen der Vorrichtung zur Bereitstellung von Dialysierflüssigkeit in stark vereinfachter schematischer Darstellung,
- Fig. 2: die Stecker-Einheit der Vorrichtung zur Bereitstellung von Dialysierflüssigkeit zusammen mit der Buchsen-Einheit der Blutbehandlungsvorrichtung oder der Vorrichtung zur Bereitstellung von Dialysierflüssigkeit von Fig. 1 in perspektivischer Darstellung,
- Fig. 3: die Buchsen-Einheit von Fig. 2 in perspektivischer Darstellung, wobei die Buchsen-Einheit für einen Spülvorgang vorbereitet ist,
- Fig. 4: die Stecker-Einheit und die Buchsen-Einheit von Fig. 2 in geschnittener Darstellung, wobei die Buchsen-Einheit zum Anschluss der Stecker-Einheit vorbereitet ist,
- Fig. 5: einen Schnitt durch die Stecker-Einheit und Buchsen-Einheit von Fig. 2, wobei die Stecker-Einheit in die Buchsen-Einheit lose eingesetzt ist, und
- Fig. 6: einen Schnitt durch die Stecker-Einheit und Buchsen-Einheit von Fig. 2, wobei die Stecker-Einheit an die Buchsen-Einheit angeschlossen ist, sodass die Strömungsverbindungen hergestellt sind, und
- Fig. 7: die Buchsen-Einheit von Fig. 2 in geschnittener Darstellung, wobei die Buchsen-Einheit für einen Spülvorgang vorbereitet ist.

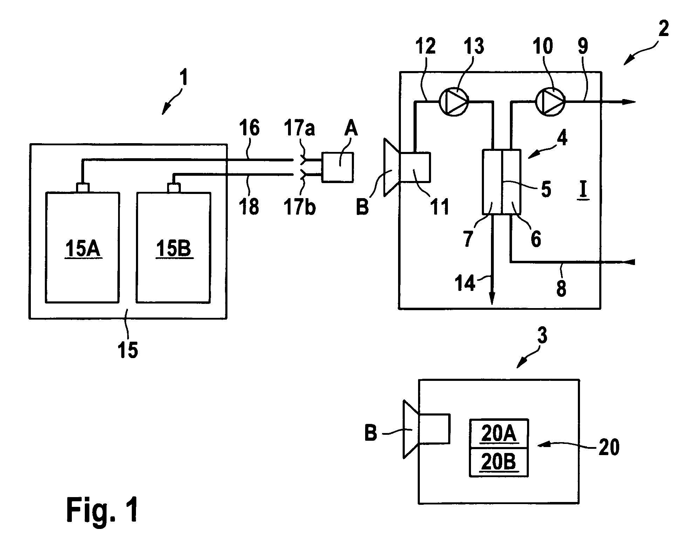

Fig. 1 zeigt in stark vereinfachter schematischer Darstellung eine Vorrichtung 1 zur Bereitstellung einer medizinischen Flüssigkeit, insbesondere Dialysierflüssigkeit, zusammen mit einer Blutbehandlungsvorrichtung 2 und einer Vorrichtung 3 zum Befüllen der Vorrichtung zur Bereitstellung von Dialysierflüssigkeit. Die Blutbehandlungsvorrichtung 2 kann eine extrakorporale Dialysevorrichtung oder eine Vorrichtung zur Peritonealdialyse sein. Bei dem vorliegenden Ausführungsbeispiel ist die Blutbehandlungsvorrichtung 2 eine Dialysevorrichtung, die über einen Dialysator 4 verfügt, der durch eine semipermeable Membran 5 in eine Blutkammer 6 und eine Dialysierflüssigkeitskammer 7 unterteilt ist. Von dem Patienten führt eine Blutzuführleitung 8 zu der Blutkammer 6 des Dialysators 4, während eine Blutrückführleitung 9, in die eine Blutpumpe 10 geschaltet ist, von der Blutkammer 6 zu dem Patienten führt. Die Blutzuführ- und -rückführleitung, 8, 9 bilden zusammen mit der Blutkammer 6 den extrakorporalen Blutkreislauf I der Dialysevorrichtung 2.

Die frische Dialysierflüssigkeit wird aus einem Dialysierflüssigkeitsreservoir 11 über eine Dialysierflüssigkeitszuführleitung 12, in die eine Dialysierflüssigkeitspumpe 13 geschaltet ist, zu der Dialysierflüssigkeitskammer 7 des Dialysators 4 geleitet, während verbrauchte Dialysierflüssigkeit über eine Dialysierflüssigkeitsabführleitung 14 aus der Dialysierflüssigkeitskammer abfließt.

Zur Bereitstellung frischer Dialysierflüssigkeit dient die Vorrichtung 1, die bei dem vorliegenden Ausführungsbeispiel zwei Beutel oder Kanister 15A und 15B aufweist. Beide Beutel oder Kanister 15A, 15B bilden eine Einheit 15, wobei der Beutel 15A vor der Dialysebehandlung mit frischer Dialysierflüssigkeit befüllt und der Beutel 15B leer ist.

Von dem Dialysierflüssigkeitsbeutel 15A führt eine Zulaufleitung 16 zu dem einen Anschluss 17a einer Stecker-Einheit A, während von dem anderen Anschluss 17b der Stecker-Einheit A eine Ablaufleitung 18 zu dem Leerbeutel 15B führt.

Die Stecker-Einheit A wird zur Bereitstellung von Dialysierflüssigkeit vor der Behandlung an eine Buchsen-Einheit B angeschlossen, die an der Blutbehandlungsvorrichtung 2 vorgesehen ist, sodass frische Dialysierflüssigkeit über die Zulaufleitung 16 dem Dialysierflüssigkeitsreservoir 10 zugeführt und verbrauchte Dialysierflüssigkeit über die Ablaufleitung 18 abgeführt werden kann. Die Dialysierflüssigkeit kann aber auch direkt dem der Dialysierflüssigkeitskammer 7 des Dialysators zugeführt werden.

Die Vorrichtung 1 zur Bereitstellung von Dialysierflüssigkeit wird an der Vorrichtung 3 mit frischer Dialysierflüssigkeit befüllt. Mit der Vorrichtung 3 zum Befüllen kann die Vorrichtung 2 zum Bereitstellen von Dialysierflüssigkeit auch entleert werden. Zur Aufnahme frischer Dialysierflüssigkeit dient ein Tank 20A und zur Aufnahme verbrauchter Dialysierflüssigkeit dient ein Tank 20B. Die erforderlichen Leitungen und Pumpen werden in der stark schematischen Darstellung nicht gezeigt.

Die Vorrichtung 3 zum Befüllen und Entleeren der Vorrichtung 1 zur Bereitstellung frischer und Aufnahme verbrauchter Dialysierflüssigkeit 1 verfügt über eine Buchsen-Einheit B, an der die Stecker-Einheit A der Vorrichtung 1 zur Bereitstellung von Dialysierflüssigkeit angeschlossen wird. Die Buchsen-Einheit B der Blutbehandlungsvorrichtung 2 und die Buchsen-Einheit B der Vorrichtung 3 zum Befüllen bzw. Entleeren können identisch oder unterschiedlich ausgebildet sein. Bei dem vorliegenden Ausführungsbeispiel sind die Buchsen-Einheiten B identisch ausgebildet. Beide Buchsen-Einheiten B sind derart ausgebildet, dass sich mit der Stecker-Einheit A der Vorrichtung 1 zur Bereitstellung von Dialysierflüssigkeit eine flüssigkeitsdichte Strömungsverbindung mit beiden Vorrichtungen 2 und 3 in beiden Richtungen für frische und verbrauchte Dialysierflüssigkeit herstellen lässt.

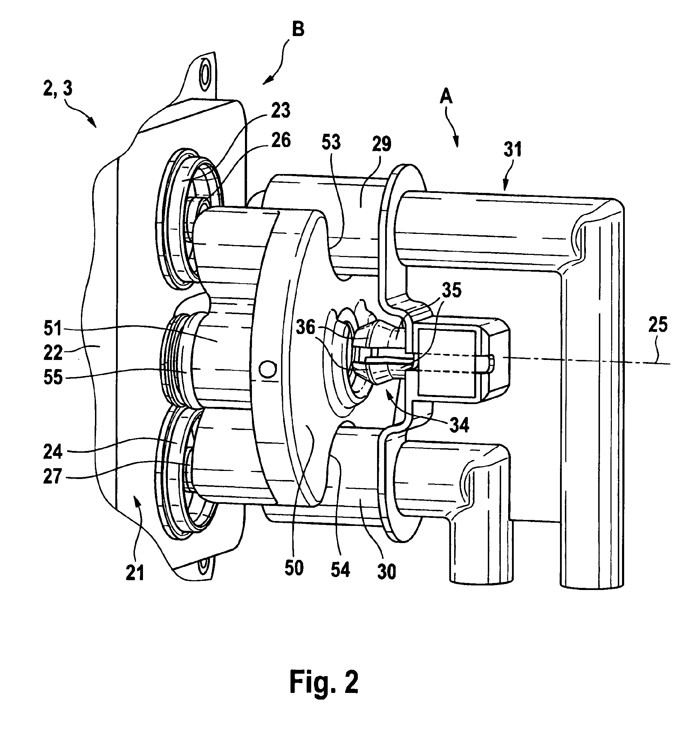

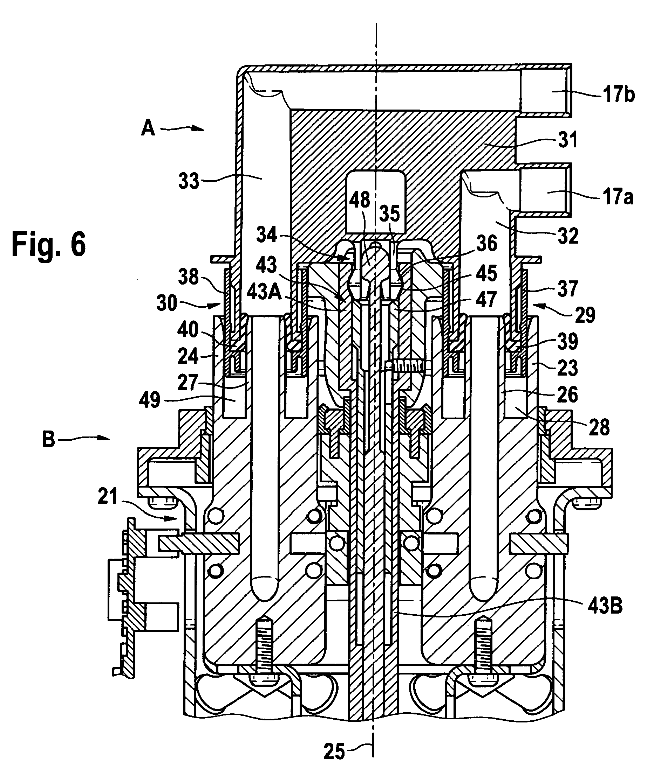

Nachfolgend wird die Stecker-Einheit A der Vorrichtung 1 zur Bereitstellung von Dialysierflüssigkeit zusammen mit der Buchsen-Einheit B unter Bezugnahme auf die Figuren 2 bis 7 im Einzelnen beschrieben.

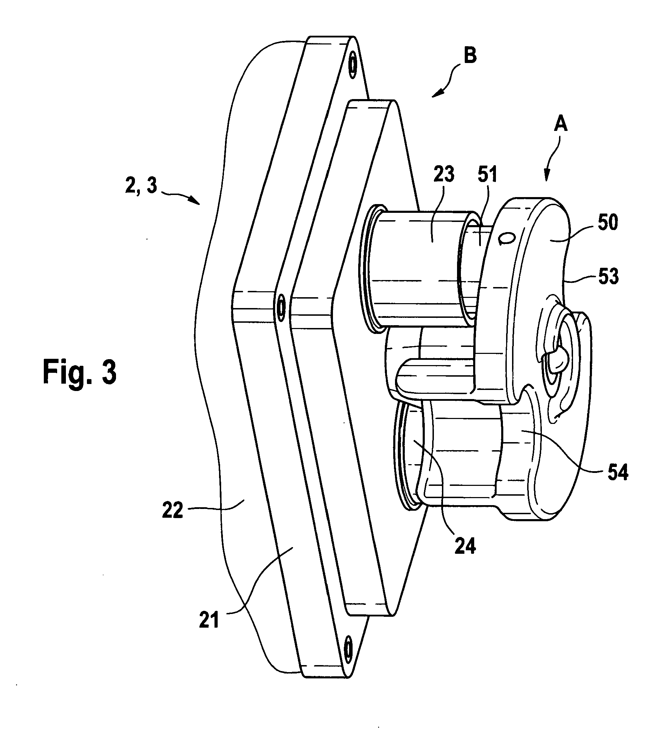

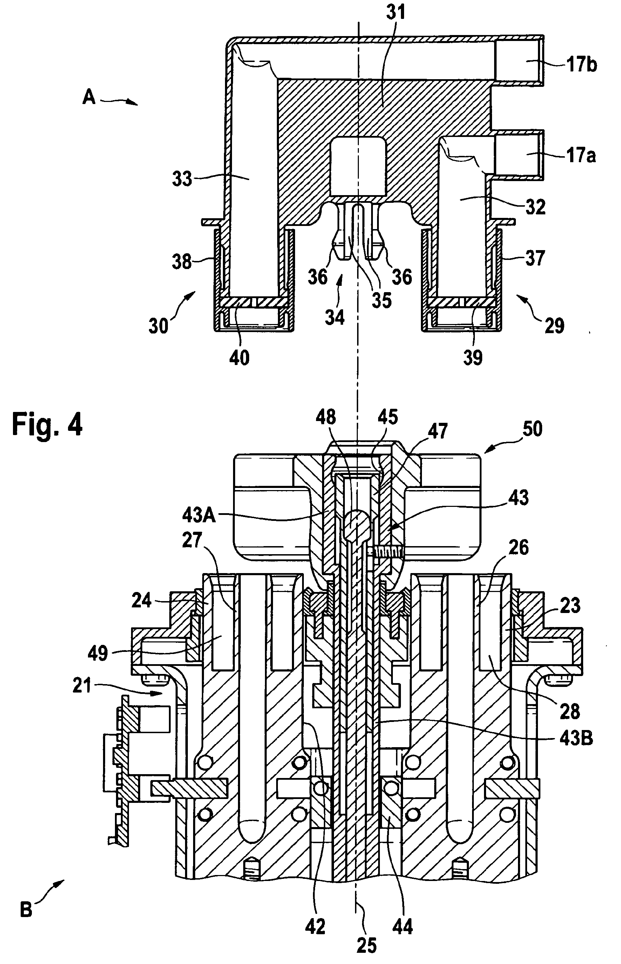

Die Figuren 2 und 3 zeigen die Stecker-Einheit A und die Buchsen-Einheit B in perspektivischer Darstellung, während die Figuren 4 bis 7 die Stecker- und Buchsen-Einheit A, B in geschnittener Darstellung zeigen.

Die Buchsen-Einheit B der Blutbehandlungsvorrichtung 2 kann Teil einer nicht dargestellten austauschbaren Behandlungskassette sein. Die Buchsen-Einheit B kann aber auch Teil einer nicht austauschbaren Einheit sein.

Fig. 2 zeigt die Buchsen-Einheit B zusammen mit der Stecker-Einheit A in perspektivischer Darstellung. Mit der Stecker-Einheit A kann die Vorrichtung 1 zur Bereitstellung von Dialysierflüssigkeit einerseits an die Vorrichtung 3 zum Befüllen und Entleeren und andererseits an die Blutbehandlungsvorrichtung 2 angeschlossen werden.

Die Buchsen-Einheit B weist einen Gehäusekörper 21 auf, der in einer Gehäusewand 22 der der Blutbehandlungsvorrichtung 2 oder der Vorrichtung 3 zum Befüllen eingesetzt ist. In dem Gehäusekörper 21 der Buchsen-Einheit B sind zwei zylindrische Anschlussteile 23, 24 vorgesehen, die in einer gemeinsamen Ebene zu beiden Seiten der zentralen Achse 25 der Buchsen-Einheit angeordnet sind. Die zylindrischen Anschlussteile 23, 24 umschließen jeweils konzentrisch ein Anschlussstück 26 bzw. 27, wobei das Anschlussstück 26 zum Zuführen frischer Dialysierflüssigkeit und das Anschlussstück 27 zum Abführen verbrauchter Dialysierflüssigkeit dient (Figuren 4 bis 7).

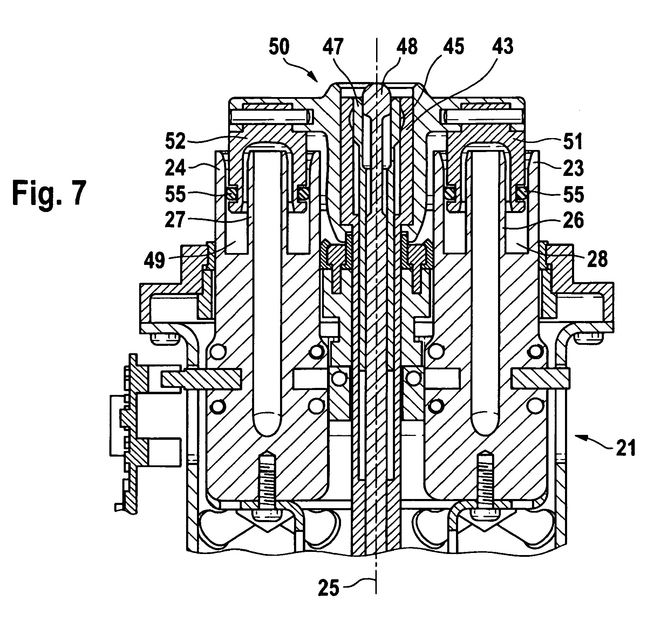

Von den beiden Anschlussteilen 23, 24 wird jeweils ein Raum umschlossen, der flüssigkeitsdicht verschlossen werden kann. Der flüssigkeitsdicht verschlossene Raum bildet eine Spülkammer 28, 49, durch die eine Spülflüssigkeit geleitet werden kann, die über nicht näher dargestellte Kanäle zuströmen oder abströmen kann (Fig. 7). Zum Spülen der Buchsen-Einheit B werden die Spülkammern 28, 49 von Spülflüssigkeit durchströmt. Dies wird nachfolgend noch im Einzelnen beschrieben.

Die Anschlussteile 23, 24 der Buchseneinheit B sind zusammen mit dem Anschlussstücken 27, 28 in dem Gehäusekörper 21 längsverschiebbar geführt, sodass die Anschlussteile und-stücke aus dem Gehäusekörper vorgeschoben bzw. in den Gehäusekörper zurückgezogen werden können. Die Antriebseinheit zum Vorschieben bzw. Zurückziehen der Anschlussteile und -stücke ist in den Figuren nicht dargestellt. Sie kann eine elektromotorische oder hydraulische Antriebseinheit sein.

Die Stecker-Einheit A (Figuren 4 bis 6) der Vorrichtung 1 zur Bereitstellung frischer und zur Aufnahme verbrauchter Dialysierflüssigkeit verfügt über entsprechende Konnektoren 29, 30, die mit den Anschlussstücken 27, 28 flüssigkeitsdicht verbunden werden. Die Stecker-Einheit A weist einen Steckerkörper 31 auf, der die beiden Konnektoren 29, 30 verbindet. Der Steckerkörper 31 weist einen Zulaufkanal 32 auf, der an dem einen Konnektor 29 angeschlossen ist, und weist einen Ablaufkanal 33 auf, der an dem anderen Konnektor 30 angeschlossen ist. An dem Anschluss 17a des Zulaufkanals 32 ist die Zulaufleitung 16 und an dem Anschluss 17b des Ablaufkanals 33 ist die Ablaufleitung 18 der Vorrichtung 1 zur Bereitstellung von frischer bzw. Aufnahme verbrauchter Dialysierflüssigkeit angeschlossen. Zwischen den beiden Konnektoren 29, 30 befindet sich ein Ansatzstück 34, mit dem eine zunächst nur lose Verbindung zwischen der Stecker-Einheit A und der Buchsen-Einheit B hergestellt werden kann.

Das Ansatzstück 34 weist mehrere umfangsmäßig verteilt angeordnete Rastelemente 35 auf, die an einem Ende des Ansatzsstücks angeformt sind. An den Außenseiten der freien Enden der Rastelemente 35 sind Rastnasen 36 ausgebildet. Die Konnektoren 29 und 30 verfügen über Berührungsschutzhülsen 37 und 38, die auf die Konnektoren 29, 30 des Steckerkörpers 31 einrastend aufgesetzt sind. Die Konnektoren 29, 30 werden jeweils von einer Membran 39, 40 verschlossen, die von den Anschlussstücken 26, 27 der Buchsen-Einheit B durchdrungen wird.

Der Gehäusekörper 21 der Buchsen-Einheit B weist eine zentrale Ausnehmung 42 auf, in der ein rohrförmiges Aufnahmestück 43 angeordnet ist, in das sich das Ansatzstück 34 der Stecker-Einheit A einsetzen lässt. Das Aufnahmestück 43 ist mit einem Lager 44 um die Achse 25 drehbar gelagert, das in die zentrale Ausnehmung 42 des Gehäusekörpers 21 eingesetzt ist. Das Aufnahmestück 43 wird mit einer nicht dargestellten Antriebseinheit gedreht.

Das rohrförmige Aufnahmestück 43 weist einen sich aus dem Gehäusekörper 21 erstreckenden vorderen Abschnitt 43A und einen sich in den Gehäusekörper erstreckenden hinteren Abschnitt 43B auf, wobei der vordere Abschnitt 43A einen größeren Außen- bzw. Innendurchmesser als der hintere Abschnitt 43B hat. An der Innenseite des vorderen Endes des vorderen Abschnitts 43A des Aufnahmestücks 43 sind umfangsgemäß verteilt angeordneten Ausnehmungen 45 vorgesehen, in die die Rastnasen 36 der Rastelemente 35 des Ansatzstücks 34 einrasten, wenn die Stecker-Einheit A lose auf die Buchsen-Einheit B aufgesetzt wird.

In dem rohrförmigen Aufnahmestück 43 ist längsverschiebbar ein als rohrförmiger Körper ausgebildetes Tastelement 47 geführt, dass mit einer nicht dargestellten Feder vorgespannt ist, sodass beim Einsetzen des Ansatzstücks 34 in das Aufnahmestück 43 das Tastelement 47 entgegen der Federspannung zurückgeschoben wird.

In dem rohrförmigen Tastelement 47 ist ein stiftförmiger Körper 48 zum Verriegeln des Ansatzstücks 34 in dem Aufnahmestück 43 geführt. Der stiftförmige Körper 48 kann mit einer nicht dargestellten Antriebseinheit in Längsrichtung vorgeschoben und wieder zurückgezogen werden, um das Ansatzstück 34 in dem Aufnahmestück 43 freizugeben bzw. zu verriegeln.

Fig. 4 zeigt die Buchsen-Einheit B in der Position, in der die Stecker-Einheit A lose auf die Buchsen-Einheit B aufgesetzt werden kann. Der stiftförmige Körper 48 ist in dem Aufnahmestück 43 zurückgezogen, sodass die Rastelemente 35 mit den Rastnasen 36 des Ansatzstücks 34 in das Aufnahmestück 43 mit den Ausnehmungen 45 einrasten können.

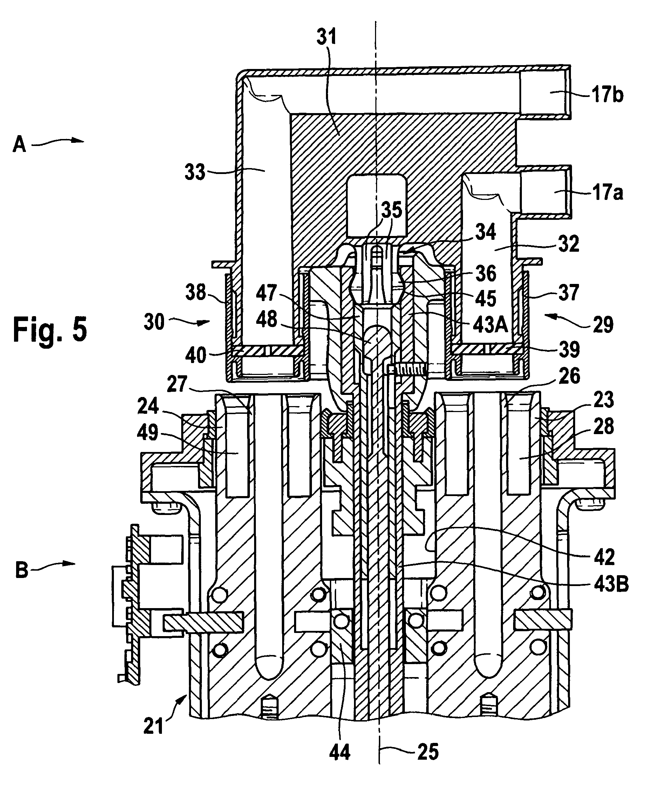

Fig. 5 zeigt die Position, in der die Stecker-Einheit A lose auf die Buchsen-Einheit B aufgesetzt ist, wobei das Ansatzstück 34 in das Aufnahmestück 43 eingerastet ist. Die Stecker-Einheit A wird dabei nur lose gehalten, ohne dass die Strömungsverbindungen hergestellt sind.

Die Stellung des Tastelements 47 wird von einer nicht dargestellten Einrichtung überwacht. Da das Tastelement 47 von dem Ansatzstück 34 zurückgeschoben ist, wird erkannt, dass die Stecker-Einheit A lose aufgesetzt ist. Wenn die Stecker-Einheit lose aufgesetzt ist, wird die nicht dargestellte Antriebseinheit in Betrieb gesetzt, wodurch der stiftförmige Körper 48 in dem Aufnahmestück 43 nach vorne geschoben wird. Dadurch wird die zunächst erst lose Verbindung zwischen Ansatzstück 34 und Aufnahmestück 43 verriegelt. Gleichzeitig werden die Anschlussteile 23, 24 mit den Anschlussstücken 26, 27 aus dem Gehäusekörper 21 nach vorne geschoben. Es ist auch möglich, dass der stiftförmige Körper 48 und die Anschlussteile 26, 27 miteinander verbunden sind und von einer Antriebseinheit gemeinsam bewegt werden.

Mit dem Verschieben der Anschlussteile 23, 24 durchstoßen die Anschlussstücke 26, 27 die Membranen 39,40 der Stecker-Einheit A, wodurch die flüssigkeitsdichten Verbindungen zwischen den Anschlussstücken und Konnektoren hergestellt wird. Da die Stecker-Einheit A nach der Verriegelung des Ansatzstücks mit dem Aufnahmestück fest auf der Buchsen-Einheit B sitzt, können die beim Verbinden von Stecker- und Buchsen-Einheit auftretenden Kräfte aufgenommen werden.

Das Lösen der Stecker-Einheit A von der Buchsen-Einheit B erfolgt in umgekehrter Reihenfolge. Hierzu werden der stiftförmige Körper 48 in dem Aufnahmestück 43 und die Anschlussteile 23, 24 mit den Anschlussstücken 26, 27 in dem Gehäusekörper 21 zurückgezogen, wodurch die Verbindung zwischen Ansatzstück 34 und Aufnahmestück 43 entriegelt und die Anschlussstücke 26, 27 aus den Konnektoren 29, 30 gezogen werden. Die Entriegelung kann gleichzeitig mit dem Zurückziehen der Anschlussstücke oder vor dem Zurückziehen der Anschlussstücke erfolgen.

Die Buchsen-Einheit B verfügt über einen Verschlusskörper 50 zum Verschluss der beiden Anschlussteile 23, 24, um einen Spülvorgang mit einer Spüllösung durchführen zu können.

Der Verschlusskörper 50 weist zwei Verschlussstücke 51, 52 auf, die im gleichen Abstand wie die Konnektoren 29, 30 der Stecker-Einheit A zueinander angeordnet sind und die gleiche Ausbildung aufweisen wie die Konnektoren der Stecker-Einheit aufweisen. Die beiden Verschlussstücke 51, 52 sind in dem Verschlusskörper 50 an ihrem rückwärtigen Ende verschlossen. An den beiden gegenüberliegenden Seiten, an denen die Konnektoren 29, 30 nicht angeordnet sind, weist der Verschlusskörper 50 halbkreisförmige Ausschnitte 53, 54 auf. Die Ausschnitte 53, 54 schließen mit den Verschlussstücken jeweils einen rechten Winkel ein.

Der Verschlusskörper 50 mit den Verschlussstücken 51, 52 ist mit dem vorderen Abschnitt 43A des Aufnahmestücks 43 der Buchsen-Einheit B verbunden. Da das Aufnahmestück 43 um die Längsachse 25 drehbar gelagert ist, kann durch Drehen des Aufnahmestücks 43 mit der nicht dargestellten Antriebseinheit auch der Verschlusskörper 50 mit den Verschlussstücken 51, 52 um die Achse 25 gedreht werden.

Fig. 2 zeigt den Verschlusskörper 50 mit den Verschlussstücken 51, 52 in der Position, in der sich die Stecker-Einheit A auf die Buchsen-Einheit B aufsetzen lässt. In dieser Position befinden sich die halbkreisförmigen Ausschnitte 53, 54 vor den Anschlussteilen 23, 24 bzw. Anschlussstücken 26, 27 der Buchsen-Einheit B, während die Verschlussstücke 51, 52 in einer Ebene angeordnet sind, die senkrecht auf der Ebene steht, in der die Anschlussteile 23, 24 angeordnet sind. In dieser Position kann die Stecker-Einheit A in die Buchsen-Einheit B gesteckt werden.

Für die Einleitung des Spülvorgangs wird der Verschlusskörper 50 mit den Konnektoren durch Drehen des Aufnahmestücks 43 von der nicht dargestellten Antriebseinheit um 90° verschwenkt, sodass sich die Verschlussstücke 51, 52 vor den Anschlussteilen 23, 24 befinden. Damit sind die Anschlussteile aber noch nicht verschlossen. Daraufhin werden die Anschlussteile 23, 24 aus dem Gehäusekörper 21 vorgeschoben, sodass die Verschlussstücke 51, 52 in die Anschlussteile 23, 24 eingeschoben werden. Damit sind die Spülkammern 28, 49 flüssigkeitsdicht verschlossen (Fig. 3, Fig. 7). Zur Abdichtung der Verschlussstücke 51, 52 gegenüber den Anschlussteilen 23, 24 können Ringdichtungen 55 vorgesehen sein. Nach Beendigung des Spülvorgangs werden die Anschlussteile wieder zurückgezogen. Der Verschlusskörper mit den Verschlussstücken kann nunmehr wieder in die Ausgangsposition zurückgedreht werden (Fig. 2).

Von Vorteil ist, dass nach der Ausrichtung der Verschlussstücke relativ zu den Anschlussteilen durch die Relativbewegung der Verschlussstücke und Anschlussteile beide Teile ineinandergreifen, so dass ein flüssigkeitsdichter Verschluss der Spülkammern gewähreistet ist.

Der Verschlusskörpers 50 stellt einen Bestandteil der Buchsen-Einheit B dar. Ein separater Stecker oder dergleichen ist daher nicht erforderlich. Die Buchsen-Einheit B erlaubt eine vollautomatische Steuerung sowohl des Anschlusses der Stecker-Einheit A an die Buchsen-Einheit B als auch der Einleitung des Spülvorgangs, sodass die Handhabung insgesamt vereinfacht wird. Da das Einsetzen der Stecker-Einheit A in die Buchsen-Einheit B erkannt wird, kann der Befüll- oder Entleervorgang automatisch eingeleitet und die Stecker-Einheit automatisch freigegeben werden. Auch kann der Spülvorgang automatisch eingeleitet und beendet werden.

## Patentansprüche

1. Medizinische Vorrichtung mit einer Buchsen-Einheit (B) zum Anschluss einer Stecker-Einheit einer Vorrichtung zur Bereitstellung von medizinischen Flüssigkeiten, wobei
die Buchsen-Einheit (B) mindestens ein Anschlussstück (26, 27) zum Anschluss mindestens eines Konnektors der Stecker-Einheit aufweist, so dass beim Anschluss des Konnektors an das Anschlussstück eine Strömungsverbindung zum Zuführen oder Abführen einer Flüssigkeit herstellbar ist, und das mindestens eine Anschlussstück (26, 27) unter Bildung einer Spülkammer (28, 49) von einem Anschlussteil (23, 24) konzentrisch umschlossen wird und die Buchsen-Einheit (B) einen Verschlusskörper (50) mit mindestens einem Verschlussstück (51, 52) zum Verschluss der mindestens einen Spülkammer aufweist,
**dadurch gekennzeichnet, dass**
der Verschlusskörper (50) um eine Drehachse (25) drehbar gelagert ist, wobei das mindestens eine Verschlussstück (51, 52) an dem Verschlusskörper (50) im Abstand zu der Drehachse angeordnet ist, und
in einer ersten Drehstellung das mindestens eine Verschlussstück (51, 52) und der mindestens eine Anschlussteil (23, 24) auf einer gemeinsamen Achse liegen, so dass durch eine Relativbewegung von Stecker-Einheit und Buchsen-Einheit (B) eine Verbindung zwischen Verschlussstück und Anschlussteil zum Verschluss der Spülkammer herstellbar ist, und
in einer zweiten Drehstellung das mindestens eine Verschlussstück (51, 52) und das mindestens eine Anschlussstück (23, 24) zueinander versetzt angeordnet sind, so dass beim Einstecken der Stecker-Einheit in die Buchsen-Einheit (B) eine Verbindung zwischen dem mindestens einen Konnektor (29, 30) der Stecker-Einheit (A) und dem mindestens einen Anschlussstück (26, 27) der Buchsen-Einheit (B) herstellbar ist.

2. Medizinische Vorrichtung nach Anspruch 1, **dadurch gekennzeichnet, dass** die Buchsen-Einheit (B) Mittel (43) zum Vorschieben des mindestens einen Anschlussstücks (23, 24) aus der Buchsen-Einheit und zum Zurückziehen des mindestens einen Anschlussstücks (23, 24) in die Buchsen-Einheit aufweist.

3. Medizinische Vorrichtung nach Anspruch 1 oder 2, **dadurch gekennzeichnet, dass** die Buchsen-Einheit (B) ein erstes Anschlussstück (23) zum Anschluss eines ersten Konnektors der Stecker-Einheit und ein zweites Anschlussstück (24) zum Anschluss eines zweiten Konnektors der Stecker-Einheit aufweist, so dass eine erste Strömungsverbindung zum Zuführen von Flüssigkeit und eine zweite Strömungsverbindung zum Abführen von Flüssigkeit herstellbar ist, und die Buchsen-Einheit (B) einen Verschlusskörper (50) mit einem ersten Verschlussstück 51) zum Verschluss der ersten Spülkammer (28) und einem zweiten Verschlussstück (52) zum Verschluss der zweiten Spülkammer (49) der Buchsen-Einheit (B) aufweist, wobei das erste und zweite Verschlussstück (51, 52) zu beiden Seiten der Drehachse (25) angeordnet sind.

4. Medizinische Vorrichtung nach einem der Ansprüche 1 bis 3, **dadurch gekennzeichnet, dass** der Verschlusskörper (50) zu beiden Seiten der Drehachse (x) angeordnete Ausschnitte (53, 54) zur Aufnahme der Konnektoren der Stecker-Einheit beim Einstecken der Stecker-Einheit in die Buchsen-Einheit aufweist.

5. Medizinische Vorrichtung nach Anspruch 4, **dadurch gekennzeichnet, dass** der zwischen den Ausschnitten (53, 54) und den Anschlussteilen (23, 24) eingeschlossene Winkel ein rechter Winkel ist.

6. Medizinische Vorrichtung nach einem der Ansprüche 1 bis 5, **dadurch gekennzeichnet, dass** die Buchsen-Einheit (B) Mittel (43, 47,48) zum lösbaren Verbinden der Stecker-Einheit und der Buchsen-Einheit aufweisen.

7. Medizinische Vorrichtung nach Anspruch 6, **dadurch gekennzeichnet, dass** die Mittel (43, 47, 48) zum lösbaren Verbinden der Stecker-Einheit und der Buchsen-Einheit ein Aufnahmestück (43) aufweisen, in das ein Ansatzstück der Stecker-Einheit einsetzbar ist.

8. Medizinische Vorrichtung nach Anspruch 7, **dadurch gekennzeichnet, dass** das Aufnahmestück (43) der Buchsen-Einheit (B, B') Ausnehmungen (45) zur Aufnahme von Rastnasen des Ansatzstücks der Stecker-Einheit aufweist.

9. Medizinische Vorrichtung nach Anspruch 7 oder 8, **dadurch gekennzeichnet, dass** in dem Aufnahmestück (43) ein stiftförmigen Körper (48) längsverschiebbar geführt ist, der in eine Ausnehmung des Ansatzstücks der Stecker-Einheit einführbar ist, so dass zur Herstellung einer Verbindung zwischen der Stecker-Einheit und der Buchsen-Einheit (B) das Ansatzstück der Stecker-Einheit aufspreizbar ist.

10. Medizinische Vorrichtung nach einem der Ansprüche 6 bis 9, **dadurch gekennzeichnet, dass** die Mittel (43, 47, 48) zum lösbaren Verbinden der Stecker-Einheit und der Buchsen-Einheit (B) Mittel (47) zum Detektieren des Ansatzstücks der Stecker-Einheit in dem Aufnahmestück (43) der Buchsen-Einheit (B) aufweisen.

11. Medizinische Vorrichtung nach Anspruch 10, **dadurch gekennzeichnet, dass** die Mittel zum Detektieren des Ansatzstücks der Stecker-Einheit ein federnd vorgespanntes Tastelement (47) aufweisen, das derart in der Buchsen-Einheit (B) angeordnet ist, dass das Tastelement beim Einführen des Ansatzstücks der Stecker-Einheit in das Aufnahmestück (43) der Buchsen-Einheit (B) entgegen der Federspannung verschiebbar ist.

12. Medizinische Vorrichtung nach einem der Ansprüche 7 bis 11, **dadurch gekennzeichnet, dass** das Aufnahmestück (43) drehbar an der Buchsen-Einheit (B) gelagert ist, wobei der Verschlusskörper (50) an dem Aufnahmestück (43) der Buchsen-Einheit befestigt ist.

13. Medizinische Vorrichtung nach einem der Ansprüche 1 bis 12, **dadurch gekennzeichnet, dass** die medizinische Vorrichtung eine Blutbehandlungsvorrichtung (2), insbesondere eine extrakorporale Dialysevorrichtung oder eine Vorrichtung zur Peritionealdialyse ist.

14. Medizinische Vorrichtung nach einem der Ansprüche 1 bis 12, **dadurch gekennzeichnet, dass** die medizinische Vorrichtung eine Vorrichtung (3) zum Befüllen einer Vorrichtung zur Bereitstellung von medizinischen Flüssigkeiten für eine Blutbehandlungsvorrichtung ist.

15. Anordnung bestehend aus einer Vorrichtung (1) zur Bereitstellung von medizinischen Flüssigkeiten für eine medizinische Vorrichtung und einer medizinischen Vorrichtung (2, 3) nach einem der Ansprüche 1 bis 12, wobei die Vorrichtung (1) zur Bereitstellung von medizinischen Flüssigkeiten eine Stecker-Einheit (A) zum Anschluss an die Buchsen-Einheit (B) der medizinischen Vorrichtung (2, 3) aufweist.

## Claims

1. Medical device comprising a socket unit (B) for connecting a plug unit of a device for providing medical fluids, the socket unit (B) comprising at least one connection piece (26, 27) for connecting at least one connector of the plug unit so that a flow connection can be produced for supplying or removing a fluid when the connector is connected to the connection piece, and the at least one connection piece (26, 27) being surrounded concentrically by a connection part (23, 24) thus forming a rinsing chamber (28, 49), and the socket unit (B) comprising a closure body (50) having at least one closure piece (51, 52) for closing the at least one rinsing chamber,
**characterised in that**
the closure body (50) is rotatably mounted about an axis of rotation (25), the at least one closure piece (51, 52) being arranged on the closure body (50) at a distance from the axis of rotation, and, in a first rotational position, the at least one closure piece (51, 52) and the at least one connection part (23, 24) lie on a common axis so that a connection between the closure piece and the connection part for closing the rinsing chamber can be produced by a relative movement of the plug unit and the socket unit (B), and, in a second rotational position, the at least one closure piece (51, 52) and the least one connection piece (23, 24) are arranged so as to be offset from one another so that a connection between the at least one connector (29, 30) of the plug unit (A) and the least one connection piece (26, 27) of the socket unit (B) can be produced when the plug unit is inserted into the socket unit (B).

2. Medical device according to claim 1, **characterised in that** the socket unit (B) comprises means (43) for advancing the at least one connection piece (23, 24) out of the socket unit and for retracting the at least one connection piece (23, 24) into the socket unit.

3. Medical device according to either claim 1 or claim 2, **characterised in that** the socket unit (B) comprises a first connection piece (23) for connecting a first connector of the plug unit and a second connection piece (24) for connecting a second connector of the plug unit so that a first flow connection can be produced for supplying fluid and a second flow connection can be produced for removing fluid, and the socket unit (B) comprises a closure body (50) having a first closure piece (51) for closing the first rinsing chamber (28) and a second closure piece (52) for closing the second rinsing chamber (49) of the socket unit (B), the first and second closure piece (51, 52) being arranged on both sides of the axis of rotation (25).

4. Medical device according to any of claims 1 to 3, **characterised in that** the closure body (50) comprises cut-outs (53, 54) arranged on both sides of the axis of rotation (x) for receiving the connectors of the plug unit when the plug unit is inserted into the socket unit.

5. Medical device according to claim 4, **characterised in that** the angle enclosed between the cut-outs (53, 54) and the connection parts (23, 24) is a right angle.

6. Medical device according to any of claims 1 to 5, **characterised in that** the socket unit (B) comprises means (43, 47, 48) for detachably connecting the plug unit and the socket unit.

7. Medical device according to claim 6, **characterised in that** the means (43, 47, 48) for detachably connecting the plug unit and the socket unit comprise a receiving piece (43), into which an extension piece of the plug unit can be inserted.

8. Medical device according to claim 7, **characterised in that** the receiving piece (43) of the socket unit (B, B') comprises recesses (45) for receiving snap-in noses of the extension piece of the plug unit.

9. Medical device according to either claim 7 or claim 8, **characterised in that** a pin-shaped body (48), which can be introduced into a recess in the extension piece of the plug unit, is guided in a longitudinally displaceable manner in the receiving piece (43) so that the extension piece of the plug unit can be splayed out in order to produce a connection between the plug unit and the socket unit (B).

10. Medical device according to any of claims 6 to 9, **characterised in that** the means (43, 47, 48) for detachably connecting the plug unit and the socket unit (B) comprise means (47) for detecting the extension piece of the plug unit in the receiving piece (43) of the socket unit (B).

11. Medical device according to claim 10, **characterised in that** the means for detecting the extension piece of the plug unit comprise a spring-loaded sensing member (47) which is arranged in the socket unit (B) in such a way that the sensing member can be moved against the spring tension when the extension piece of the plug unit is introduced into the receiving piece (43) of the socket unit (B).

12. Medical device according to any of claims 7 to 11, **characterised in that** the receiving piece (43) is rotatably mounted on the socket unit (B), the closure body (50) being fastened to the receiving piece (43) of the socket unit.

13. Medical device according to any of claims 1 to 12, **characterised in that** the medical device is a blood treatment device (2), in particular an extracorporeal dialysis device or a device for peritoneal dialysis.

14. Medical device according to any of claims 1 to 12, **characterised in that** the medical device is a device (3) for filling a device for providing medical fluids for a blood treatment device.

15. Arrangement consisting of a device (1) for providing medical fluids for a medical device and a medical device (2, 3) according to any of claims 1 to 12, wherein the device (1) for providing medical fluids comprises a plug unit (A) for connecting to the socket unit (B) of the medical device (2, 3).

## Revendications

1. Dispositif médical avec une unité femelle (B) pour le branchement d'une unité mâle d'un dispositif pour la mise à disposition de liquides médicaux,
l'unité femelle (B) comprenant au moins une pièce de raccordement (26, 27) pour le branchement d'au moins un connecteur de l'unité mâle, de façon à ce que, lors du branchement du connecteur à la pièce de connexion, une liaison d'écoulement puisse être établie pour l'introduction ou l'évacuation d'un liquide et l'au moins une pièce de raccordement (26, 27) étant entourée de manière concentrique par une partie de connexion (23, 24) en formant une chambre de rinçage (28, 49) et l'unité femelle (B) comprenant un corps de fermeture (50) avec au moins une pièce de fermeture (51, 52) pour la fermeture de l'au moins une chambre de rinçage,
**caractérisé en ce que**
le corps de fermeture (50) est monté tournant autour d'un axe de rotation (25), l'au moins une pièce de fermeture (51, 52) étant disposée sur le corps de fermeture (50) à une certaine distance de l'axe de rotation, et
dans une première position de rotation, l'au moins une pièce de fermeture (51, 52) et l'au moins une partie de connexion (23, 24) se trouvent sur un axe commun, de façon à ce qu'un mouvement relatif de l'unité mâle et de l'unité femelle (B) permette une liaison entre la pièce de fermeture et la partie de connexion pour la fermeture de la chambre de rinçage, et
dans une deuxième position de rotation, l'au moins une pièce de fermeture (51, 52) et l'au moins une pièce de connexion (23, 24) sont disposées de manière décalée entre elles, de façon à ce que lors de l'enfichage de l'unité mâle dans l'unité femelle (B), une liaison puisse être établie entre l'au moins un connecteur (29, 30) de l'unité mâle (A) et l'au moins une pièce de raccordement (26, 27) de l'unité femelle (B).

2. Dispositif médical selon la revendication 1, **caractérisé en ce que** l'unité femelle (B) comprend des moyens (43) pour le coulissement de l'au moins une pièce de connexion (23, 24) hors de l'unité femelle et pour le retour de l'au moins une pièce de connexion (23, 24) dans l'unité femelle.

3. Dispositif médical selon la revendication 1 ou 2, **caractérisé en ce que** l'unité femelle (B) comprend une première pièce de connexion (23) pour le branchement d'un premier connecteur de l'unité mâle et une deuxième pièce de connexion (24) pour le branchement d'un deuxième connecteur de l'unité mâle, de façon à ce qu'une première liaison d'écoulement pour l'introduction d'un liquide et une deuxième liaison d'écoulement pour l'évacuation d'un liquide puissent être établies, et l'unité femelle (B) comprend un corps de fermeture (50) avec une première pièce de fermeture (51) pour la fermeture de la première chambre de rinçage (28) et une deuxième pièce de fermeture (52) pour la fermeture de la deuxième chambre de rinçage (49) de l'unité femelle (B), la première et la deuxième pièce de fermeture (51, 52) étant disposées des deux côtés de l'axe de rotation (25).

4. Dispositif médical selon l'une des revendications 1 à 3, **caractérisé en ce que** le corps de fermeture (50) comprend des découpes (53, 54) disposées des deux côtés de l'axe de rotation (25) pour le logement des connecteurs de l'unité mâle lors de l'enfichage de l'unité mâle dans l'unité femelle.

5. Dispositif médical selon la revendication 4, **caractérisé en ce que** l'angle formé entre les découpes (53, 54) et les parties de connexion (23, 24) est un angle droit.

6. Dispositif médical selon l'une des revendications 1 à 5, **caractérisé en ce que** l'unité femelle (B) comprend des moyens (43, 47, 48) pour la liaison amovible de l'unité mâle et de l'unité femelle.

7. Dispositif médical selon la revendication 6, **caractérisé en ce que** les moyens (43, 47, 48) comprennent, pour la liaison amovible de l'unité mâle et de l'unité femelle, une pièce de logement (43) dans laquelle une pièce rapportée de l'unité mâle peut être insérée.

8. Dispositif médical selon la revendication 7, **caractérisé en ce que** la pièce de logement (43) de l'unité femelle (B, B') comprend des évidements (45) pour le logement d'embouts d'encliquetage de la pièce rapportée de l'unité mâle.

9. Dispositif médical selon la revendication 7 ou 8, **caractérisé en ce qu'**un corps en forme de tige (48) pouvant être introduit dans un évidement de la pièce rapportée de l'unité mâle est guidé longitudinalement de manière coulissante dans la pièce de logement (43), de façon à ce que, pour l'établissement d'une liaison entre l'unité mâle et l'unité femelle (B), la pièce rapportée de l'unité mâle puisse être élargie.

10. Dispositif médical selon l'une des revendications 6 à 9, **caractérisé en ce que** les moyens (43, 47, 48) comprennent, pour la liaison amovible de l'unité mâle et de l'unité femelle (B), des moyens (47) pour la détection de la pièce rapportée de l'unité mâle dans la pièce de logement (43) de l'unité femelle (B).

11. Dispositif médical selon la revendication 10, **caractérisé en ce que** les moyens de détection de la pièce rapportée de l'unité mâle comprennent un élément palpeur (47) précontraint de manière élastique, qui est disposé dans l'unité femelle (B) de façon à ce que l'élément palpeur puisse coulisser, lors de l'introduction de la pièce rapportée de l'unité mâle dans la pièce de logement (43) de l'unité femelle (B), à l'encontre de la tension du ressort.

12. Dispositif médical selon l'une des revendications 7 à 11, **caractérisé en ce que** la pièce de logement (43) est logée de manière rotative au niveau de l'unité femelle (B), le corps de fermeture (50) étant fixé à la pièce de logement (43) de l'unité femelle.

13. Dispositif médical selon l'une des revendications 1 à 12, **caractérisé en ce que** le dispositif médical est un dispositif de traitement du sang (2), plus particulièrement un dispositif de dialyse extracorporelle ou un dispositif de dialyse péritonéale.

14. Dispositif médical selon l'une des revendications 1 à 12, **caractérisé en ce que** le dispositif médical est un dispositif (3) pour le remplissage d'un dispositif pour la mise à disposition de liquides médicaux pour un dispositif de traitement du sang.

15. Système constitué d'un dispositif (1) pour la mise à disposition de liquides médicaux pour un dispositif médical et d'un dispositif médical (2, 3) selon l'une des revendications 1 à 12, le dispositif (1) de mise à disposition de liquides médicaux comprenant une unité mâle (A) pour le branchement à l'unité femelle (B) du dispositif médical (2, 3).
